(19) Europäisches Patentamt
European Patent Office
Office européen des brevets

(11) Publication number: **0 464 206 A1**

(12) **EUROPEAN PATENT APPLICATION published in accordance with Art. 158(3) EPC**

(21) Application number: **90904947.0**

(22) Date of filing: **23.03.90**

(86) International application number: **PCT/JP90/00392**

(87) International publication number: **WO 90/11080 (04.10.90 90/23)**

(51) Int. Cl.5: **A61K 31/70**, //C07H19/213

(30) Priority: **24.03.89 JP 72371/89**

(43) Date of publication of application: **08.01.92 Bulletin 92/02**

(84) Designated Contracting States: **AT BE CH DE DK ES FR GB IT LI LU NL SE**

(71) Applicant: **DAIICHI PHARMACEUTICAL CO., LTD.**
**14-10, Nihonbashi 3-chome**
**Chuo-ku, Tokyo 103(JP)**

(72) Inventor: **KITAOKA, Hirofumi 14-21, Nankodai 5-chome**
**Izumi-ku**
**Sendai-shi Miyagi 981(JP)**
Inventor: **KAWAJIRI, Shinichi Daiichi Pharmaceutical Co., Ltd. Research Institute 16-13, Kitakasai 1-chome Edogawa-Ku Tokyo 134(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **DRUG FOR IMPROVING BRAIN FUNCTION.**

(57) A drug containing as the active ingredient adenosine 3',5'-cyclic monophosphate of formula (1) or a salt thereof, wherein $R_1$ is hydrogen, lower alkyl or lower acyl, $R_2$ is hydrogen or aralkylthio, and $R_3$ is hydrogen or lower acyl. It is effective in treating cerebrovascular dementia, dementias represented by Alzheimer disease, mental symptom disorders represented by depression, nervous symptom disorders represented by Parkinson disease, degenerative or regressive diseases of the central nervous system, brain disorders due to cerebral ischemia or hemorrhage represented by cerebral infarction, brain disorders due to traumas such as contusion, etc.

EP 0 464 206 A1

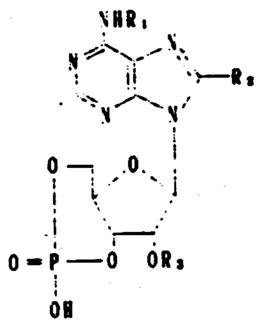

(I)

## TECHNICAL FIELD

This invention relates to a nootropic agent containing an adenosine-3',5'-cyclic monophosphate derivative (hereinafter abbreviated as cAMP derivative) represented by the following formula (I):

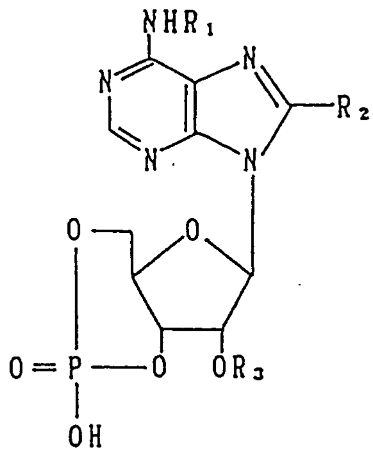

(I)

wherein $R_1$ represents a hydrogen atom, a lower alkyl group or a lower acyl group; $R_2$ represents a hydrogen atom or an aralkylthio group; and $R_3$ represents a hydrogen atom or a lower acyl group, or a salt thereof as an active ingredient.

## BACKGROUND ART

Examples of diseases accompanied by a decline in brain function include cerebral disorders, dementia, neuropathy and psychopathy caused by, for example, cerebral atrophy or cerebral infarct. Recently patients suffering from these diseases are rapidly increasing. Examples of drugs to be applied to the treatment of these diseases include Idebenone, Vinpocetin, Calcium hopatenate, Inderoxazine, and dopamine. Although these drugs show each some clinical characteristics, any of them does not have fully satisfactory clinical effects.

It is known that the compound of formula (I) is effective in the treatment of, for example, shock and dermal ulcer. However, these diseases never relate to the above-mentioned diseases accompanied by a decline in brain function.

The present inventors have conducted extensive studies in order to found out a drug sufficiently effective on the above-mentioned diseases, thus completing the present invention.

## DISCLOSURE OF INVENTION

The present invention relates to a nootropic agent which containing a cAMP derivative of formula (I) or a salt thereof as an active ingredient.

In formula (I), the lower alkyl group include methyl, ethyl, n-propyl, isopropyl, n-butyl, tert-butyl groups and the like. The lower acyl group include acetyl, propanoyl, butanoyl, pentanoyl groups and the like. Examples of the aralkyl group include benzyl, phenylethyl, phenylpropyl groups and the like.

Salt of the cAMP derivative of formula (I) include acid addition salts formed with inorganic acids such as hydrochloric acid and sulfuric acid, acid addition salts formed with organic acids such as oxalic acid and succinic acid, and a salt formed from a phosphate group and an alkali metal or alkaline earth metal such as sodium, potassium, calcium, magnesium, etc.

Further, the preferred examples of the cAMP derivative of formula (I) and its salt include sodium $N^6$-2'-0-dibutyryladenosine-3',5'-cyclic phosphate, sodium adenosine-3',5'-cyclic phosphate, sodium $N^6$-monobutyryladenosine-3',5'-cyclic phosphate, sodium 2-0'-monobutyryladenosine-3',5'-cyclic phosphate, $N^6$-butyl-8-benzylthioadenosine-3',5'-cyclic phosphate, and 8-benzylthioadenosine-3',5'-cyclic phosphate.

Examples of the diseases accompanied by a decline in brain function relating to the present invention include cerebrovascular dementia, dementia such as Alzheimer's disease, psychopathy such as depression, neuropathy such as Parkinson's disease, degenerative or regressive disease in central nervous system,

cerebral disorders caused by cerebral hemorrhage or cerebral ischemia such as cerebral infarct and cerebral disorders caused by trauma such as bruise. It is expected that dementia, Parkinson's disease and cerebral disorders caused by cerebral ischemia or cerebral hemorrhage, among these diseases, can be effectively treated by the administration of the cAMP derivative of formula (I) or a salt thereof.

The cAMP derivative of formula (I) and a salt thereof may be formulated into various dosage forms (for example, tablet, capsule, granules, injection) together with additives (for example, filler, disintegrator, stabilizer, binder) by a known pharmaceutical techniques.

The cAMP derivative of formula (I) and a salt thereof may be either orally or parenterally (for example, intravenous injection) administered. In the case of intravenous drip infusion, it may be usually given to an adult in a dose of from 5 to 200 µg/kg/min.

The cAMP derivative of formula (I) and a salt thereof were administered to rats in order to examine the acute toxicities of these compounds. As a result, it has been proved that they are highly safe.

## BEST MODE FOR CARRYING OUT INVENTION

The present invention is further explained in detail hereinafter by the following Examples, but the present invention is not limited to these examples.

### Example 1

300 mg of sodium $N^6$-2'-0-dibutyryladenosine-3',5'-cyclic phosphate (hereinafter, abbreviated as DBcAMP) was dissolved in a saline and administered to five patients, who suffered from cerebrovascular dementia or Alzheimer's type senile dementia with Parkinson's disease as a complication, by intravenous drip infusion for approximately 8 hours per day continuously.

Each patient showed remarkable encephalatrophy in computed tomography (hereinafter, abbreviated as CT) and had suffered from serious disorientation or amnesia for 1 to 6 years. These patients gave no response to a call and their intellectual level was decreased. Further, they had been kept laying with slight spontaneous movement of their limbs and showed incontinence of urine, which suggested the lowered mental level and conscious level. Therefore, it was recognized that these patients suffered from serious dementia.

Moreover, the administration of available psychotropic agents or cerebral metabolic agents to these patients does not achieve any effect.

The results are shown in Table 1 below.

TABLE 1

Test 1

| | |
|---|---|
| Name: | T. T |
| Sex: | Male |
| Age: | 85 |
| Diagnosis: | Alzheimer's type dementia (with Parkinson's disease and bedsore as complications) |
| Symptom and condition just before administration (including observations on CT and brain wave): | Kept laying for 5 years. Neither speech nor response to call. Serious dementia. Remarkable rigidity in the limbs and oily skin on the face. Almost a vegetable. Extremely difficult to treat bedsore. Remarkable encephalatrophy and enlarged ventricle in CT. |
| Progress (including therapeutics) before administration: | Use of cerebral metabolism activator caused no change. |
| Administration of actosin (dose/period): | Starting from Sep. 8, 1988, administered 300 mg/day of DBcAMP with intravenous drip infusion for 8 hours up to this date. |
| Progress after administration and evaluation (symptom/ condition): | Observed rapid turn of face to name call and speech "Yes" or "Good Morning" to call 1 week after administration. Said "Stop" when shaken. Oil skin disappeared. Mysclerosis in right arm reduced, facilitating drip infusion. Improvements in various symptoms caused by a decline in brain function, compared before the administration. |
| Side effect: | None. |

TABLE 1 (cont'd)

Test 2

| | |
|---|---|
| Name: | K. Y |
| Sex: | Female |
| Age: | 86 |
| Diagnosis: | Cerebrovascular dementia (cerebral infarct 8 years before. Sequelae. Acute renal insufficiency as complication) |
| Symptom and condition just before administration (including observations on CT and brain wave): | Kept laying, with right-half paralysis. Serious depression in volition. Neither any speech nor response to call for about 2 months.<br>Enlarged ventricle and subdural effusion in low-absorption area in left middle cerebral artery in CT. |
| Progress (including therapeutics) before administration: | Use of cerebral metabolism activator and cerebro-vasodilator caused no change. |
| Administration of actosin (dose/period): | From Dec. 2, 1988 to Jan. 13, 1989, administered 300 mg/day of DBcAMP by intravenous drip infusion for 8 hours. |
| Progress after administration and evaluation (symptom/ condition): | Showed "Yes" or "No" for simple question by shaking head 1 week after the administration. No speech but groan.<br>Remarkable improvement in symptoms caused by a decline in brain function, compared before the administration. |
| Side effect: | None. |

TABLE 1 (cont'd)

Test 3

| | |
|---|---|
| Name: | C. S |
| Sex: | Male |
| Age: | 72 |
| Diagnosis: | Cerebrovascular dementia (with Parkinson's disease as complication) |
| Symptom and condition just before administration (including observations on CT and brain wave): | Kept laying for 4 years, almost a vegetable. No response to call. Obviously encephalatrophy, enlarged ventricle and low-absorption area around ventricle in CT. |
| Progress (including therapeutics) before administration: | Use of anti-Parkinson's agent and cerebrovascular activator caused no change. |
| Administration of actosin (dose/period): | From Sep. 22 to Sep. 26, 1988, administered 300 mg/day of DBcAMP by intravenous drip infusion for 8 hours. |
| Progress after administration and evaluation (symptom/ condition): | Groaned in night from the second day of the administration, after a year's silence. Groaning became larger thereafter. Improvement in various symptoms caused by a decline in brain function. |
| Side effect: | None. |

TABLE 1 (cont'd)

Test 4

| | |
|---|---|
| Name: | K. S |
| Sex: | Female |
| Age: | 79 |
| Diagnosis: | Alzheimer's type dementia (with Parkinson's disease as complication) |
| Symptom and condition just before administration (including observations on CT and brain wave): | Kept laying for 6 years, showing no response to call. Serious dementia. Remarkable trepidation in limbs, myosclerosis and oral dyskinesia. Obvious encephalatrophy and enlarged ventricle but no infarct in CT. |
| Progress (including therapeutics) before administration: | Attempted to improve nutritional state, because of systemic bad conditions. Use of anti-Parkinson's agent and cerebral metabolism improver caused no change. |
| Administration of actosin (dose/period): | From Aug. 30 to Sep. 11, 1988, administered 300 mg/day of DBcAMP by intravenous drip infusion for 8 hours. |
| Progress after administration and evaluation (symptom/condition): | Answered to call 1 week after the administration. Soliloquized in night. Remarkable improvement in symptoms caused by a decline in brain function. |
| Side effect: | None. |

TABLE 1 (cont'd)

Test 5

| | |
|---|---|
| Name: | K. K. |
| Sex: | Male |
| Age: | 88 |
| Diagnosis: | Cerebrovascular dementia, multiple cerebral infarct (with chronic obturation plumonary diseases as complications) |
| Symptom and condition just before administration (including observations on CT and brain wave): | Kept laying with incontinence of urine. Scarcely responded to call from 3 years ago. Serious dementia.<br>Obvious encephalatrophy, small infarction nests around ventricle and low-absorption region basal ganglia. |
| Progress (including therapeutics) before administration: | Use of cerebral metabolism activator caused no change. |
| Administration of actosin (dose/period): | From Sep. 5, 1988, administered 300 mg/day of DBcAMP by intravenous drip infusion for 8 hours up to this date. |
| Progress after administration and evaluation (symptom/condition): | Responded call from the third day of the administration. Answered simple question 1 month after the administration (for example, "One year has 365 days." "One day has 24 hours.", "One hour has 60 minutes.") Capable of having simple conversation, slowly. Capable of sitting on bed. Highly motivated. Conscious desire to urinate made vesical training possible, requiring no balloon catheter for incontinence of urine.<br>Remarkable improvement in symptoms caused by a decline in brain function. |
| Side effect: | None. |

As clearly showed in the above Table 1, remarkable amelioration such as responding a call, giving a speech or attempting to rise were observed 3 to 14 days after the administration of DBcAMP. Further, the continuous administration caused extremely remarkable improvements including mental improvements such as telling one's name, recognizing one's family and realizing time and place; improvements in side symptoms of dementia such as disappearance of urinary incontinence, depression in volition and neuropathy; and improvements in major symptoms of dementia such as enhancement of intellectual and mental functions. Furthermore, the administration of DBcAMP resulted in the disappearance of oily skin and reduced myosclerosis, thus improving the symptoms of the complicated Parkinson's disease. Therefore, it was clinically proved that DBcAMP is useful as a nootropic agent since it can improve various symptoms caused by a decline in brain function.

Example 2

DBcAMP was intravenously injected into rats and mice to thereby examine the acute toxicity thereof. As a result, $LD_{50}$ of this compound to rat was 459 mg/kg while that to mouse was 545 mg/kg.

INDUSTRIAL APPLICABILITY

The cAMP derivative of formula (I) and a salt thereof exerted excellent clinical effect on a decline in brain function. Thus these compounds are effective in the treatment of cerebrovascular dementia, dementia such as Alzheimer's disease, psychopathy such as depression, neuropathy such as Parkinson's disease, degenerative or regresive disease in central nervous system, cerebral disorders caused by cerebral hemorrhage or cerebral ischemia such as cerebral infarct and cerebral disorders caused by trauma such as bruise.

Therefore, the cAMP derivative of formula (I) and a salt thereof are excellent as a nootropic agent.

## Claims

**1.** A nootropic agent which containing an adenosine-3',5'-cyclic monophosphate derivative represented by the following formula (I):

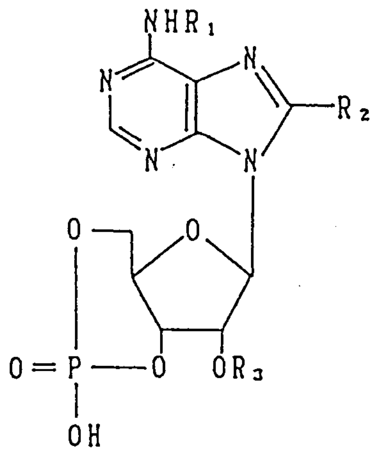

(I)

wherein $R_1$ represents a hydrogen atom, a lower alkyl group or a lower acyl group; $R_2$ represents a hydrogen atom or an aralkylthio group; and $R_3$ represents a hydrogen atom or a lower acyl group,
or a salt thereof as an active ingredient.

# INTERNATIONAL SEARCH REPORT

International Application No PCT/JP90/00392

**I. CLASSIFICATION OF SUBJECT MATTER** (if several classification symbols apply, indicate all) [6]

According to International Patent Classification (IPC) or to both National Classification and IPC

Int. $Cl^5$ A61K31/70//C07H19/213

**II. FIELDS SEARCHED**

Minimum Documentation Searched [7]

| Classification System | Classification Symbols |
|---|---|
| IPC | A61K31/70, C07H19/16 - 19/213 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 52-156935 (Daiichi Seiyaku Co., Ltd.), 27 December 1977 (27. 12. 77), (Family: none) | 1 |
| A | JP, A, 62-252726 (Hoechst Japan Ltd.), 4 November 1987 (04. 11. 87) & EP, A, 253962 | 1 |
| A | Chemical Abstracts. Vol 109 (No.25): 221676w, 1988 & Acta Med. Rom., 26(1), p.52-5, 1988 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 6, 1990 (06. 06. 90) | June 18, 1990 (18. 06. 90) |
| **International Searching Authority** | **Signature of Authorized Officer** |
| Japanese Patent Office | |